# EUROPEAN PATENT SPECIFICATION

(11) **EP 0 981 374 B1**
(45) Date of publication and mention of the grant of the patent: **21.11.2007**
(21) Application number: 98922348.2
(22) Date of filing: 18.05.1998
(51) Int. Cl.: A61K 47/36, A61K 9/06

(54) **SUSTAINED-RELEASE DELAYED GELS**
ZEITVERZÖGERTE GELZUSAMMENSETZUNGEN MIT VERLÄNGERTER WIRKSTOFFVERABREICHUNG
GELS A ACTION DIFFEREE ET A LIBERATION PROLONGEE

(30) Priority: 16.05.1997 US 857973; 15.08.1997 US 912902
(43) Date of publication of application: 01.03.2000
(73) Proprietor: AMGEN INC., Thousand Oaks, CA 91320-1799 (US)
(72) Inventor: GOLDENBERG, Merrill, Seymour, Thousand Oaks, CA 91360 (US); BEEKMAN, Alice, C., Thousand Oaks, CA 91361 (US); GU, Jian, Hua, Thousand Oaks, CA 91360 (US)
(74) Representative: Brown, John D.
(86) International application number: PCT/US1998/010013
(87) International publication number: WO 1998/051348

(56) References cited:
- EP-A- 0 506 191
- US-A- 4 241 099
- DATABASE WPI Week 8907 Derwent Publications Ltd., London, GB; AN 89-049865 [07] XP002082332 & JP 01 005460 A (SAN-EI CHEM. IND. LTD.,JP) 10 January 1989
- DATABASE WPI Week 9139 Derwent Publications Ltd., London, GB; AN 91-284799 [39] XP002082333 & JP 03 188149 A ((SAKA) OTSUKA SEIYAKU KOGYO KK,JP) 16 August 1991

## Description

### FIELD OF THE INVENTION

The present invention relates to sustained-release formulations using alginate delayed gels and methods thereof.

### BACKGROUND

With the advances in genetic and cell engineering technologies, the availability of recombinant proteins has engendered advances in the use of proteins as medicaments for therapeutic applications. Many illnesses or conditions treated with pharmaceutical proteins require sustained protein levels to achieve the most effective therapeutic result. However, as with most protein pharmaceuticals, the generally short biological half-life requires frequent administration. These repeated injections are given at various intervals which result in fluctuating medication levels at a significant physical and monetary burden on the patients. Since many conditions respond better to controlled levels of a pharmaceutical, a need exists for controlled release of a medicament to provide longer periods of consistent release. Such sustained-release medicaments would provide the patient with not only enhanced prophylactic, therapeutic or diagnostic effects, but also a decrease in the frequency of injections as well as in overall costs.

Current attempts to sustain medication levels in humans or animals between doses have included the use of biodegradable polymers as matrices to control medicament release. For example, Great Britain Patent No. 1,388,580 discloses the use of hydrogels for sustained-release of insulin. U.S. Patent No. 4,789,550 discloses the use of polylysine coated alginate microcapsules for delivery of protein by encapsulating living cells. Sustained-release attempts have also utilized anionic or cationic polymer compositions surrounded by ionic polymers of the opposite charge for encapsulating cells capable of producing biologically active compositions. U.S. Patent No. 4,744,933. Likewise, multiple coats of anionic or cationic cross-linking polymers have also been disclosed as means for obtaining controlled release. U.S. Patent Nos. 4,690,682 and 4,789,516. In addition, further attempts disclose the use of alginates alone, or alginates coated with other biodegradable polymers, for controlled release of polypeptide compositions or cation precipitates thereof. PCT WO 96/00081, PCT WO 95/29664 and PCT WO 96/03116.

These attempts, however, have provided insufficient means for obtaining sustained-release delivery of desired protein pharmaceuticals. It is generally known that the use of certain biodegradable polymers, e.g., polylactide co-glycolide, under in vivo conditions, exhibit high initial bursts of medicament release. Johnson, O. et al., Nature Med., 2/7: 795 (1996). Furthermore, it is generally known that proteins used with current forms of sustained-release preparations can undergo denaturation and lose their bioactivity upon exposure to the encapsulating agents. Such preparations use organic solvents which can have deleterious effects on the protein of choice. Finally, as discussed below, use of alginate alone has not provided the desired controlled protein release necessary for effective therapeutic results.

In general, alginates are well known, naturally occurring, anionic, polysaccharides comprised of 1,4-linked-β-D-mannuronic acid and α-L-guluronic acid. Smidsrod, O. et al., Trends in Biotechnology, 8: 71-78 (1990); Aslani, P. et al., J. Microencapsulation, 13 5: 601-614 (1996). Alginates typically vary from 70% mannuronic acid and 30% guluronic acid, to 30% mannuronic acid and 70% guluronic acid. Smidsrod, supra. Alginic acid is water insoluble whereas salts formed with monovalent ions like sodium, potassium and ammonium are water soluble. McDowell, R.H., "Properties of Alginates" (London, Alginate Industries Ltd., 4th edition 1977). Polyvalent cations are known to react with alginates to spontaneously form gels.

Alginates have a wide variety of applications such as food additives, adhesives, pharmaceutical tablets, template for new cell growth, and wound dressings. Alginates have also been recommended for protein separation techniques. For example, Gray, C.J. et al., in Biotechnology and Bioengineering, 31: 607-612 (1988) entrapped insulin in zinc/calcium alginate gels for separation of insulin from other serum proteins.

Alginate matrices have also been well documented for drug delivery systems, see for example U.S. Patent No. 4,695,463 disclosing an alginate based chewing gum delivery system and pharmaceutical preparations. Alginate beads have been used for controlled release of various proteins such as: tumor necrosis factor receptor in cation-alginate beads coated with polycations, Wee, S.F, Proceed. Intern. Symp. Control. Rel. Bioact. Mater., 21: 730-31 (1994); transforming growth factor encapsulated in alginate beads, Puolakkainen, P.A. et al., Gastroenterology, 107 : 1319-1326 (1994) ; angiogenic factors entrapped in calcium-alginate beads, Downs, E.C. et al., J. of Cellular Physiology, 152: 422-429 (1992); albumin entrapped in chitosan-alginate microcapsules, Polk, A. et al., J. Pharmaceutical Sciences, 83/2: 178-185 (1994), or chitosan-calcium alginate beads coated with polymers, Okhamafe, A. O. et al., J. Microencapsulation, 13/5: 497-508 (1996); hemoglobulin encapsulated with chitosan-calcium alginate beads, Huguet, M.L. et al., J. Applied Polymer Science, 51: 1427-1432 (1994), Huguet, M.L. et al., Process Biochemistry, 31: 745-751 (1996); and interleukin-2 encapsulated in alginate-chitosan microspheres, Liu, L.S. et al., Proceed. Intern. Symp. Control. Rel. Bioact. Mater, 22: 542-543 (1995).

Systems using alginate gel beads, or alginate/calcium gel beads, to entrap proteins suffer from lack of any sustained-release effect due to rapid release of the protein from the alginate beads. Liu, L. et al., J. Control. Rel., 43: 65-74 (1997). To avoid such rapid release, a number of the above systems attempt to use polycation polymer coatings (e.g., polylysine, chitosan) to retard the release of the protein alginate beads. See, e.g., Wheatley, M.A. et al., J. Applied Polymer Science, 43: 2123-2135 (1991); Wee, S.F. et al. supra ; Liu, L.S. et al. supra ; Wee, S.F. et al., Controlled Release Society, 22: 566-567 (1995) and Lim, et al. supra.

Polycations, such as polylysine, are positively charged polyelectrolytes which interact with the negatively charged alginate molecules to form polyelectrolyte complexes that act as diffusion barriers on the bead surface. Problems can occur with the use of polycations in that: (1) such formulations may be cytotoxic due to the polycations (Huguet, M.L. et al., supra ; Zimmermann, Ulrich, Electrophoresis, 13: 269 (1992); Bergmann, P. et al., Clinical Science, 67: 35 (1984)); (2) polycations are prone to oxidation; (3) beads with polycation coatings tend not to be erodible and build up in the body; (4) such formulations are made via laborious coating procedures which include multiple coatings of the polycation polylysine (Padol, et al., Proceed. Intern. Symp. Control. Rel. Bioact. Mater, 2: 216 (1986) and (5) ionic interactions between the protein and the polycations can result in loss of protein activity or cause protein instability.

In addition to the above systems, there also exist delivery systems which gel after injection, or are organic based and/or thixotropic based. Gelled depots which gel after injection in the body can sustain the release of trapped drugs. These would include the temperature induced gelation of poloxamers, (e.g., Pluronics^{®}, U.S. Patent No. 2,741,573). These are liquid at cold temperatures but gel at elevated temperatures such as body temperatures. These systems are difficult to control due to variations in ambient temperature conditions, and variations in anatomical temperatures especially if subcutaneous injections are used.

As for organic based gelation systems, such systems are not suited for fragile protein drugs that are destabilized in the presence of organic solvents or non-physiological conditions. Thixotropic gels of aluminum stearate in oils have also been used to prolong the activity of penicillin. Buckwalter, et al, J. Am. Pharm Assoc., 137: 472 (1948); Thompson, R.E., American Journal Clinical Nutrition, 7: 311 (1959); Thompson, Robert E., Sustained Release of Parenteral Drugs, Bulletin of Parenteral Drug Assoc., 14: 6-17, (1960); Chen, Y., Journal of Parenteral Science & Tech., 35: 106 (1981). JP01 005 460 A discloses gelling gellan gum using an alkaline metal salt and an acidic substance. Proteins tend to be destabilized in the presence of these type of oil containing systems.

Sustained-release drug delivery systems involving a controlled time delay for gelation in the body are not generally known in the art. These type of delayed gel systems are, however, known in the context of plant tissue culture, immobilization of cells, microsphere formation, insecticide release and the food industry. For example, alginates have been used with insoluble calcium complexes and δ-gluconolactone as a proton donor for release of calcium ions into the solution for gelation. See Draget, et al, Appl Microbiol. Biotechnol, 31 : 79-83 (1989). Likewise, similar systems have been used for alginate bead formation by emulsification/internal gelation. Alginate microspheres were produced using alginate dispersed within vegetable oil and gelification initiated by reducing pH to release calcium from the insoluble complex. See Poncelet, D. et al, Appl. Microbiol. Biotechnol, 43: 644-650 (1995). Alginate systems have also been used to immobilize cells. Burke, C. discloses, in Methods of Enzymology, 135: 175-189 (1987), that dicalcium phosphate and δ-gluconolactone can be used with alginates for delayed gelation of cells. U.S. Patent No. 4,053,627 discloses the use of alginate gel discs to control release of an insecticide in an aqueous environment. These aforementioned uses are not designed for gelled depots in the body for the sustained-release of biologically active agents, especially proteins.

Accordingly, a need exists to develop delayed gel pharmaceutical formulations which achieve a better means of sustained-release for clinical applications. Numerous recombinant or natural proteins could benefit from constant long term release through delayed gel formulations and thereby provide more effective clinical results.

The present invention provides such advances. Delayed gel pharmaceutical compositions of the present invention are capable of providing protein protection, decreased degradation and slow dissolving with increased protein stability and potency. Also, pharmaceutical compositions of the present invention provide a simple, rapid and inexpensive means of controlled recombinant protein release for effective prophylactic, therapeutic or diagnostic results.

### SUMMARY OF THE INVENTION

The present invention relates to sustained-release formulations using alginate delayed gels, and methods thereof. In particular, the formation of the sustained-release delayed gels includes thixotropic alginate gels with a biologically active agent. This approach provides an advantage of producing efficient and high loading of biologically active agent within the alginate delayed gel for sustained-release delivery while achieving protein protection, decreased degradation, increased stability and potency of the agent to be delivered. Furthermore, timed gelation provides more control over the gelling properties and administration thereof.

Accordingly, one aspect of the present invention provides a sustained-release delayed gel composition, comprising a hydrophilic polymer; a biologically active agent and at least one bound polyvalent metal ion. The rate of gelation is controlled by the free calcium level, i.e., unbound polyvalent metal ion. The biologically active agent can be in a complexed form. The formation of complexed molecules and any related complexing agents are well known to those skilled in the art. In addition, the above composition may further contain polyvalent metal ion which is a mixture of bound and unbound polyvalent metal ions. Due to the time controlled nature of these delayed gels, these mixtures can be placed in the body where they can gel after injection. In addition, due to the thixotropic nature of the composition in the gel state, these mixtures can be injected while in the gel state, e.g., by pressure from the syringe, where upon they can regel in the body.

Another aspect of the present invention provides a sustained-release delayed gel composition, comprising a hydrophilic polymer; a biologically active agent; at least one bound polyvalent metal ion and further comprising at least one proton donor capable of freeing the bound polyvalent metal ion. The release of the proton from the proton donor releases the cation from the bound polyvalent metal ion.

Another aspect provides for methods to produce the sustained-release delayed gel compositions of the present invention. One method comprises the steps of mixing a biologically active agent and a hydrophilic polymer with a solvent to form a first mixture and mixing the first mixture with at least one bound polyvalent metal ion to form a second mixture. An alternative method comprises the steps of mixing a biologically active agent and a hydrophilic polymer with a solvent to form a first mixture; mixing the first mixture with at least one bound polyvalent metal ion to form a second mixture; and mixing with the second mixture at least one proton donor capable of releasing the bound polyvalent metal ion. The bound polyvalent metal ion and the proton donor can also be added to the first mixture together, or the proton donor can be added to the first mixture prior to the bound polyvalent metal ion. In addition, a step for isolating the sustained-release delayed gel composition is also contemplated.

As used herein the term bound polyvalent metal ion refers to polyvalent metal ion in a salt or chelate form or ion complex. Bound polyvalent metal ion can include a mixture of bound and unbound polyvalent metal ion. This would include as noted above release of the bound to unbound polyvalent metal ion. As used herein, the term proton donor capable of releasing bound polyvalent metal ion refers to strong acids, weak acids, or material capable of generating an acid, e.g., lactones or esters (by aqueous hydrolysis), or a poorly soluble acid or slowly dissolving acid such as adipic acid.

As used herein, the term solvent refers to aqueous or nonaqueous based solvents capable of dispersing or dissolving the biologically active agents, hydrophilic polymers, polyvalent metal ions, proton donors or complexing agents of choice. Such solvents are well known to one skilled in the art. Additions to form the first mixture and the second mixture can be done by methods well known to one skilled in the art, including but not limited to pouring and agitating, droplet addition, dispersion, spraying or mixing by using spray jets, air jets, atomizing, and electric fields. The term dispersion for purposes of this invention can mean a liquid, solid or gaseous dispersions. As used herein, the term isolating, refers to the process for isolation of the sustained-release delayed gel composition of the present invention. Such isolation and purification procedures are well known in the art.

In yet another aspect, the present invention provides for a sustained-release delayed gel composition produced by the above methods. Further aspects include delayed gel pharmaceutical formulations of the above compositions in a pharmaceutically acceptable carrier, or adjuvant. In addition, the delayed gel composition can be contained in a syringe.

In yet another aspect, the present invention provides for methods of treating indications with sustained-release delayed gel compositions containing desired biologically active agents.

### DETAILED DESCRIPTION OF THE INVENTION

### Compositions

The composition uses alginates and derivatives thereof, that can be obtained from various commercial, natural or synthetic sources well known in the art.

Likewise, bound polyvalent metal ions can be obtained from various commercial, natural or synthetic sources which are all well known in the art. Bound or sequestered polyvalent metal ions within the scope of this invention include but are not limited to manganese, strontium, iron, magnesium, calcium, barium, copper, aluminum or zinc. Metal ions can be obtained from soluble salts of a complexing agent, phosphates, tartrates, sulfates, carbonates, hydroxides, or fatty acid anions, such as oleates, thereof. One skilled in the art will appreciate other various bound polyvalent metal ions/complexes that are within the scope of the invention. Bound polyvalent metal ions can include a mixture of bound and unbound polyvalent metal ions.

Proton donors as used herein, refers to material that can generate acids. Proton donors are capable of releasing a bound-or sequestered polyvalent metal ion. Proton donors are well known in the art, and include but are not limited to lactones such as gluconolactones, esters, buffers and other slowly dissolving acids. As used herein slowly dissolving acids refer to acids such as solid acids that are of low solvent solubility. In addition, slowly dissolving acids include devices or coated materials that slowly release acids. Well known acids within the scope of the art include acetic, adipic, citric, fumaric, gluconic, lactic, malic, phosphoric and tartaric. One skilled in the art will appreciate other various proton donors that are within the scope of the invention.

As used herein, the term buffer or buffer solution refers to the use of.inorganic or organic acids or bases or a combination thereof to prepare a buffer solution as known in the art. These also include amphoteric materials or amino acids. Inorganic acids within the scope of the present invention include hydrogen halide (e.g., hydrochloric acid), phosphoric, nitric or sulfuric. Other inorganic acids would be well known to one skilled in the art and are contemplated herein. Organic acids within the scope of the invention include aliphatic carboxylic acids and aromatic acids such as formic, carbonic, acetic, propionic, butyric, valeric, caproic, acrylic, malonic, succinic, glutaric, adipic, maleic, fumaric, glycine or phenol sulfonic. Other organic acids would be well known to one skilled in the art. Organic bases include TRIS®, pyridine, PIPES®, and HEPES®. Amino acid buffers include glycine and glycine/phosphoric acid mixtures.

As used herein, biologically active agents refers to recombinant or naturally occurring proteins, whether human or animal, useful for prophylactic, therapeutic or diagnostic application, as well as non-protein based agents, such as small molecules, oligonucleotides, and inorganic or organic agents. The biologically active agent can be natural, synthetic, semi-synthetic or derivatives thereof. In addition, biologically active agents of the present invention can be precipitable. A wide range of biologically active agents are contemplated. These include but are not limited to hormones, cytokines, hematopoietic factors, growth factors, antiobesity factors, trophic factors, anti-inflammatory factors, and enzymes (see also U.S. Patent No. 4,695,463 for additional examples of useful biologically active agents). One skilled in the art will readily be able to adapt a desired biologically active agent to the compositions of present invention.

Such proteins would include but are not limited to interferons (see, U.S. Patent Nos. 5,372,808, 5,541,293 4,897,471, and 4,695,623 hereby incorporated by reference including drawings), interleukins (see, U.S. Patent No. 5,075,222, hereby incorporated by reference including drawings), erythropoietins (see, U.S. Patent Nos. 4,703,008, 5,441,868, 5,618,698 5,547,933, and 5,621,080 hereby incorporated by reference including drawings), granulocyte-colony stimulating factors (see, U.S. Patent Nos. 4,810,643, 4,999,291, 5,581,476, 5,582,823, and PCT Publication No. 94/17185, hereby incorporated by reference including drawings), stem cell factor (PCT Publication Nos. 91/05795, 92/17505 and 95/17206, hereby incorporated by reference including drawings), and the OB protein (see PCT publication Nos. 96/40912, 96/05309, 97/00128, 97/01010 and 97/06816 hereby incorporated by reference including figures). In addition, biologically active agents can also include but are not limited to anti-obesity related products, insulin, gastrin, prolactin, adrenocorticotropic hormone (ACTH), thyroid stimulating hormone (TSH), luteinizing hormone (LH), follicle stimulating hormone (FSH), human chorionic gonadotropin (HCG), motilin, interferons (alpha, beta, gamma), interleukins (IL-1 to IL-12), tumor necrosis factor (TNF), tumor necrosis factor-binding protein (TNF-bp), brain derived neurotrophic factor (BDNF), glial derived neurotrophic factor (GDNF), neurotrophic factor 3 (NT3), fibroblast growth factors (FGF), neurotrophic growth factor (NGF), bone growth factors such as osteoprotegerin (OPG), insulin-like growth factors (IGFs), macrophage colony stimulating factor (M-CSF), granulocyte macrophage colony stimulating factor (GM-CSF), megakaryocyte derived growth factor (MGDF), keratinocyte growth factor (KGF), thrombopoietin, platelet-derived growth factor (PGDF), colony simulating growth factors (CSFs), bone morphogenetic protein (BMP), superoxide dismutase (SOD), tissue plasminogen activator (TPA), urokinase, streptokinase and kallikrein. The term proteins, as used herein, includes peptides, polypeptides, consensus molecules, analogs, derivatives or combinations thereof.

Derivatives of biologically active agents may include the attachment of one or more chemical moieties to the protein moiety. Chemical modification of biologically active agents has been found to provide additional advantages under certain circumstances, such as increasing the stability and circulation time of the therapeutic protein and decreasing immunogenicity. One skilled in the art will be able to select the desired chemical modification based on the desired dosage, circulation time, resistance to proteolysis, therapeutic uses and other considerations. Derivatives such as polyethylene glycol and Fc fusion proteins are desirable.

### Complexes

The biologically active agent may be in complexed forms. This would include precipitated forms, structured forms, and association with other molecules. These complexed forms of the agent can decrease the diffusion rate of the agent out of the gel and hence sustain the agent release. These complexed forms include but are not limited to biologically active agent complexed with antibodies, substrates, receptors, lipids, polymers, and precipitants.

For example the biologically active agents, analog or derivative may be administered complexed to a binding composition. Such binding composition in addition to the benefits above may also have the effect of prolonging the circulation time of the agent, analog or derivative or enhancing the activity of the biologically active agent. Such composition may be a protein (or synonymously, peptide), derivative, analog or combination or a non-protein agent.

By way of illustration, a binding protein for the OB protein is OB protein receptor or portion thereof, such as a soluble portion thereof. Other binding proteins may be ascertained by examining OB protein, or the protein of choice, in serum, or be empirically screening for the presence of binding. Such binding will typically not interfere with the ability of OB protein or analog or derivative to bind to endogenous OB protein receptor and/or effect signal transduction. In addition to the OB protein, binding complexes will also be applicable to other therapeutic proteins of the present invention as well. Those well skilled in the art will be able to ascertain appropriate binding proteins for use with the present invention.

Likewise, precipitating agents used to precipitate the biologically active agent can be obtained from various commercial, natural or synthetic sources which are well known in the art. Precipitating agents include but are not limited to polyvalent metal ions or their salts such as acetates, citrates, chlorides, carbonates, hydroxides, oxalates, tartrates or hydroxides thereof, acids or water soluble polymers. In particular, the metal ions can include but are not limited to aluminum, barium, calcium, iron, manganese magnesium, strontium and zinc. Preferably the metal ion is zinc or the salts thereof, like acetate chloride salts. Water soluble small molecules and salts can also be used such as ammonium sulfate, acetone, ethanol and glycerol.

As for water soluble polymers these include but are not limited to polyethylene glycol, ethylene glycol/propylene glycol copolymers, hydroxyethylcellulose, amylose, carboxylmethylcellulose, dextran, polyvinyl alcohol, polyvinyl pyrolidone, poly-1, 3-dioxolane, poly-1,3,6-trioxane, ethylene/maleic anhydride copolymers, polyaminoacids, dextran, poly (n-vinyl pyrolidone) polyethylene glycol, propylene glycol homopolymers, polypropylene oxide/ethylene oxide copolymers, polyoxyethylated polyols, polyvinyl alcohol succinate, glycerine, ethylene oxides, propylene oxides, poloxamers, alkoxylated copolymers, water soluble polyanions, derivatives or combinations thereof. The water soluble polymer may be of any molecular weight, and may be branched or unbranched. For example, the preferred molecular weight of polyethylene glycol is between about 700 Da and about 100 kDa for ease in handling and efficiency of precipitation.

Other sizes and types of precipitating agents, may be used, depending on the desired therapeutic profile (e.g., the duration of sustained-release desired, the effects, if any on biological activity, the ease in handling, the degree or lack of antigenicity and other known effects of a desired precipitating agent to a therapeutic protein or analog). One skilled in the art will appreciate other precipitating agents that are within the scope of the invention.

In addition, the compositions of the present invention may also include extra excipients necessary to stabilize the biologically active agent and/or the hydrophilic polymer. These can be contained in the buffer and may include but are not limited to preservatives.

### Pharmaceutical Compositions

The sustained-release pharmaceutical compositions of the present invention may be administered by oral (e.g., liquid preparations allowing gelation in the stomach or intestines) and non-oral preparations (e.g., intramuscular, subcutaneous, transdermal, visceral, IV (intravenous), IP (intraperitoneal), intraarticular, placement in the ear, ICV (intracerebralventricular), IP (intraperitoneal), intraarterial, intrathecal, intracapsular, intraorbital, injectable, pulmonary, nasal, rectal, and uterine-transmucosal preparations). In general, comprehended by the invention are sustained-release delayed gel pharmaceutical compositions comprising effective amounts of protein, or derivative products, with the sustained-release compositions of the invention together with pharmaceutically acceptable diluents, preservatives, solubilizers, emulsifiers, adjuvants and/or carriers needed for administration. (See PCT 97/01331 hereby incorporated by reference.) The optimal pharmaceutical formulation for a desired biologically active agent will be determined by one skilled in the art depending upon the route of administration and desired dosage. Exemplary pharmaceutical compositions are disclosed in Remington's Pharmaceutical Sciences (Mack Publishing Co., 18th Ed., Easton, PA, pgs. 1435-1712 (1990)).

Due to the thixotropic nature of the delayed gel formulation, syringes can be used to administer subcutaneously. The composition may be gelled in a syringe for later injection. This gelation can be performed in a time-delayed manner. The timing is controlled by the judicious adjustment of the quantity of the gelling agent, and the proton donor if used, as well as the particle size of the polyvalent metal ion and the temperature of the mixture. Such a preparation would be used for later re-gelation in the body after injection. The term thixotropic as used herein refers to the viscosity of the gel mixture which decreases under pressure, e.g., from the syringe plunger, at which point the mixture can flow, e.g., through the syringe needle, and then reform a gel at the injection site.

The concept of delayed gelation can also be applied to filling a syringe where a sustained-release gel composition is filled in a syringe and at a preset time gels in the syringe, e.g., from a few minutes to many hours after filling. This avoids the problem of filling a syringe with material that has already gelled. These prefilled syringes can be stored for later injection into patients.

Components that may be needed for administration include diluents or buffers of various pH and ionic strength (e.g., Tris-HCl, acetate); additives such as surfactants and solubilizing agents (e.g., Tween 80, HCO-60, Polysorbate 80), lipids, liposomes, anti-oxidants (e.g., ascorbic acid, glutathione, sodium metabisulfite), additional polysaccharides (e.g., carboxymethylcellulose, sodium alginate, sodium hyaluronate, protamine sulfate, polyethylene glycol), preservatives (e.g., Thimersol, benzyl alcohol, methyl paraben, propyl paraben) and building substances (e.g., lactose, mannitol); incorporation of the material with particulate preparations of polymeric compounds such as polylactic/polyglycolic acid polymers or copolymers, etc. or combined with liposomes. Hylauronic acid may also be used as an administration component and this may have the effect of promoting even further the sustained duration in the circulation. Additionally, sustained-release compositions of the present invention may also be dispersed with oils (e.g., sesame oil, corn oil, vegetable), or a mixture thereof with a phospholipid (e.g., lecitin), or medium chain fatty acid triglycerides (e.g., Miglyol 812) to provide an oily suspension. The compositions of the present invention may also be dispersed with dispersing agents such as water-soluble polysaccharides (e.g., mannitol, lactose, glucose, starches), hyaluronic acid, glycine, fibrin, collagen and inorganic salts (e.g., sodium chloride).

In addition, also contemplated for use in the administration of the sustained-release compositions of the present invention are mechanical devices designed for pulmonary delivery of therapeutic products, including but not limited to nebulizers, metered dose inhalers, and powder inhalers, all of which are familiar to those skilled in the art.

The administration components may influence the physical state, stability, rate of in vivo release, and rate of in vivo clearance of the present proteins and derivatives. One skilled in the art will appreciate the appropriate administration components and/or the appropriate mechanical devices to use depending on the therapeutic use, route of administration, desired dosage, circulation time, resistance to proteolysis, protein stability and other considerations.

### Methods of Use

Therapeutic. Therapeutic uses depend on the biologically active agent used. One skilled in the art will readily be able to adapt a desired biologically active agent to the present invention for its intended therapeutic uses. Therapeutic uses for such agents are set forth in greater detail in the following publications hereby incorporated by reference including drawings. Therapeutic uses include but are not limited to uses for proteins like interferons (see, U.S. Patent Nos. 5,372,808, 5,541,293 4,897,471, and 4,695,623 hereby incorporated by reference including drawings), interleukins (see, U.S. Patent No. 5,075,222, hereby incorporated by reference including drawings), erythropoietins (see, U.S. Patent Nos. 4,703,008, 5,441,868, 5,618,698 5,547,933, and 5,621,080 hereby incorporated by reference including drawings), granulocyte-colony stimulating factors (see, U.S. Patent Nos. 4,999,291, 5,581,476, 5,582,823, 4,810,643 and PCT Publication No. 94/17185, hereby incorporated by reference including drawings), stem cell factor (PCT Publication Nos. 91/05795, 92/17505 and 95/17206, hereby incorporated by reference including drawings), and the OB protein (see PCT publication Nos. 96/40912, 96/05309, 97/00128, 97/01010 and 97/06816 hereby incorporated by reference including figures).

In addition, therapeutic uses of the present invention include uses of biologically active agents including but not limited to anti-obesity related products, insulin, gastrin, prolactin, adrenocorticotropic hormone (ACTH), thyroid stimulating hormone (TSH), luteinizing hormone (LH), follicle stimulating hormone (FSH), human chorionic gonadotropin (HCG), motilin, interferons (alpha, beta, gamma), interluekins (IL-1 to IL-12), tumor necrosis factor (TNF), tumor necrosis factor-binding protein (TNF-bp), brain derived neurotrophic factor (BDNF), glial derived neurotrophic factor (GDNF), neurotrophic factor 3 (NT3), fibroblast growth factors (FGF), neurotrophic growth factor (NGF), bone growth factors such as osteoprotegerin (OPG), insulin-like growth factors (IGFs), macrophage colony stimulating factor (M-CSF), granulocyte macrophage colony stimulating factor (GM-CSF), megakaryocyte derived growth factor (MGDF), keratinocyte growth factor (KGF), thrombopoietin, platelet-derived growth factor (PGDF), colony simulating growth factors (CSFs), bone morphogenetic protein (BMP), superoxide dismutase (SOD), tissue plasminogen activator (TPA), urokinase, streptokinase and kallikrein. The term proteins, as used herein, includes peptides, polypeptides, consensus molecules, analogs, derivatives or combinations thereof. In addition, the present compositions may also be used for manufacture of one or more medicaments for treatment or amelioration of the conditions the biologically active agent is intended to treat.

Combination Therapies. The present compositions and methods may be used in conjunction with other therapies, such as altered diet and exercise. Other medicaments, such as those useful for the treatment of diabetes (e.g., insulin, and possibly amylin), cholesterol and blood pressure lowering medicaments (such as those which reduce blood lipid levels or other cardiovascular medicaments), activity increasing medicaments (e.g., amphetamines), diuretics (for liquid elimination), and appetite suppressants. Such administration may be simultaneous or may be in seriatim. In addition, the present methods may be used in conjunction with surgical procedures, such as cosmetic surgeries designed to alter the overall appearance of a body (e.g., liposuction or laser surgeries designed to reduce body mass, or implant surgeries designed to increase the appearance of body mass). The health benefits of cardiac surgeries, such as bypass surgeries or other surgeries designed to relieve a deleterious condition caused by blockage of blood vessels by fatty deposits, such as arterial plaque, may be increased with concomitant use of the present compositions and methods. Methods to eliminate gall stones, such as ultrasonic or laser methods, may also be used either prior to, during or after a course of the present therapeutic methods. Furthermore, the present methods may be used as an adjunct to surgeries or therapies for broken bones, damaged muscle, or other therapies which would be improved by an increase in lean tissue mass.

### Dosages

One skilled in the art will be able to ascertain effective dosages by administration and observing the desired therapeutic effect. The dosage of the sustained-release preparation is the amount necessary to achieve the effective concentration of the biologically active agent in vivo, for a given period of time. The dosage and the preferred administration frequency of the sustained-release preparations varies with the type of the biologically active agent, the desired duration of the release, the target disease, desired administration frequency, the subject animal species and other factors. Preferably, the formulation of the molecule will be such that between about 0.10 ug/kg/day and 100 mg/kg/day will yield the desired therapeutic effect.

The effective dosages may be determined using diagnostic tools over time. By way of example, the present invention provides the dosages of OB protein. For example, a diagnostic for measuring the amount of OB protein in the blood (or plasma or serum) may first be used to determine endogenous levels of OB protein. Such diagnostic tool may be in the form of an antibody assay, such as an antibody sandwich assay. The amount of endogenous OB protein is quantified initially, and a baseline is determined. The therapeutic dosages are determined as the quantification of endogenous and exogenous OB protein (that is, protein, analog or derivative found within the body, either self-produced or administered) is continued over the course of therapy. For example, a relatively high dosage may be needed initially, until therapeutic benefit is seen, and then lower dosages used to maintain the therapeutic benefits.

### Materials and Methods

Materials. Alginate in the form of alginate salt can be found from sources, or be prepared by methods, well known in the art. Leptin, GCSF and consensus interferon are from Amgen Inc. Other chemicals are from sources well known in the art.

Delayed Gel Preparation-Introduction. The delayed gel is prepared by combining a biologically active agent and anionic polymer (e.g., alginate) mixture with a polyvalent cation salt (e.g., CaCO3) and a proton donor (e.g., acidified buffer or slowly-releasing or dissolving acid source such as δ-gluconolactone), if used. For all cases, the anionic polymer, protein and any precipitants/excipients can be prepared as one mixture. Gelation is initiated by the addition of the polyvalent cation salt and proton source, if used, to this mixture. The addition of proton, polyvalent metal ion to the polymer biologically active agent mixture can be simultaneous, or separately with proton donor first or polyvalent metal ion first. For buffer-induced gelation the polyvalent cation salt and proton source may be mixed as an aqueous suspension well in advance of the time of gelation. After gelation is started, syringes are filled before gelation occurs (typically 5 to 10 minutes). Injections can be performed before the material gels for in situ gelation, or after the material gels.

Protein-Alginate Mixture. A mixture of solvent and precipitants/excipients ( *e.g.*, zinc salts, buffers, etc.) is prepared. In rapid succession, a solution of the protein (e.g., leptin in 10 mM Tris HCl, pH 8) and sterile alginate (e.g., autoclaved 10% solution) are rapidly mixed. Where the biologically active agent is prepared as a fine suspension (e.g., zinc-leptin is typically formed at 10-15 mg/mL), it may be desirable to concentrate the suspension.

Calcium Salt. The calcium salt can be prepared as an autoclaved suspension of fine powder in water (e.g., 9.1% CaCO₃ in water).

Proton Source. For buffer-induced gels, the calcium salt suspension can be combined with buffer, such as 1 M Tris HCl, pH 7.0 or 0.5 M PIPES, pH 6.7.

For slowly dissolving or slowly releasing acid sources (δ-gluconolactone), a given weight of powder is dissolved in water at a selected time shortly before use (e.g., one minute before mixing). A preweighed mixture of dry, sterile powders of the acid source and calcium salt can also be used to simplify the process.

Solvent. The solvent can be aqueous, nonaqueous or mixtures thereof. Examples of nonaqueous solvents are dimethyl sulfoxide, dimethyl formamide, glycerol, polyethylene glycols, Pluronics^{®} and so on.

Gelation. The gelation of the polymer-drug mixture can be initiated by adding calcium salt. Temperature of the mixture, and amount and particle size of the salt can be used to control the speed of gelation. If additionally using a proton donor, the gelation of the mixture can be inititated by adding either 1) calcium salt and acidified buffer suspension (separately or together), 2) calcium salt suspension followed by a fresh solution/suspension of a slowly releasing proton source or vice versa, or 3) powders of calcium salt and proton source together or separately. After addition of the calcium salt, other precipitants/excipients ( e.g., zinc salts, buffers, etc.) can be added to the mixture. After thorough and rapid mixing, the gel mixture can be drawn up in a syringe before it gels.

Gel Loading. In general, protein loading is known. Unknown gel loadings can be determined as follows.

Burst Method. About 0.1 - 0.2 mL (exact weight taken) of gel is cast in an Eppendorf tube, then dissolved in 1 mL of 0.1M sodium citrate. The mixture is incubated at room temperature with gentle agitation until the gel disintegrates (generally 2 hours to overnight). After the resulting suspension is centrifuged at 8K rpm for 2 min. (Eppendorf, 5415 C), the absorbance at 280 nm of the supernatant is taken. Any residual solids are dissolved in 1 mL of 7M urea; the absorbance of this solution is recorded. From these absorbances the milligrams of protein per gram of gel can be calculated.

Cumulative Method. This method is used in conjunction with the in vitro release studies. The amount of protein released from the gel including the burst at the end of the study is totaled. For details, see the section on In Vitro Release Studies.

In Vitro Release Studies. The gel is either formed in an Eppendorf tube ("cast" samples) or in the syringe then extruded into the Eppendorf tube ("extruded" samples). In general, about 0.1-0.2 mL of gel is cast or extruded (the exact amount weighed). The release is begun by adding buffer (10 mM Tris HCl, pH 8 for leptin, pH 7.4 for GCSF) to each tube and placing it in an incubator shaker (New Brunswick Scientific) at 37°C and 100-200 rpm. At selected time intervals, the sample is removed from the incubator. If the gel is intact, the supernatant is removed and centrifuged (Eppendorf, 8000 rpm, 2 min) and the supernatant collected. Any solids are suspended in 1 mL fresh Tris buffer and returned to the original release tube for resumption of the release. If the gel is largely disrupted, the tube contents are centrifuged, the supernatant collected and the solids resuspended in 1 mL buffer for resumption of the release. The supernatant "timepoints" may require further centrifugation (8000 -13,000 rpm, 8 minutes) to clarify them for UV scanning. The amount of protein released is determined from the absorbance of the supernatant. After the final release sample is taken the amount left in the bead is determined by the Burst Method (above). The percent released at a given time is determined from the cumulative protein released expressed as a fraction of either the original protein load in the gel (known from the weight of gel and how it is formulated) or the final total protein released (including the final citrate-urea burst).

### In Vivo Studies.

Mouse Weight Loss. Six to eight week old female mice, type C57/BLC are obtained from Charles River and Taconic Inc. They typically weigh 20 grams. Each dose group consists of five mice. Injections are subcutaneous.

Rat PK Study. Male rats are used in this study and they typically weigh 250-300 grams. The injections are performed in a similar manner to that described in the mouse weight loss experiments. Blood is sampled by catheter collection at various time intervals post injection and the samples analyzed for leptin by an ELISA assay.

### EXAMPLES

The following examples are offered to more fully illustrate the invention, but are not to be construed as limiting the scope thereof. In addition, with respect to the above disclosure or the examples below, one skilled in the art will be able to make the necessary changes to the disclosures for large scale production.

### Example 1

This in vivo example shows that leptin in a buffer-induced delayed gel is active and exhibits sustained release in comparison to a solution of leptin. This example also illustrates a system that gels after injection into the animal. A sterile calcium carbonate suspension was prepared from sieved solids of less than 75 micron particle size. A premix of this with Tris pH 7 was added to leptin in alginate (in 10 mM Tris pH 8) such that the final concentrations of the ingredients were: 2% alginate (sterile filtered), 10 mg/mL leptin, 7 mM Tris pH 8, 150 mM Tris pH 7 and 24 mM CaCO3 (assuming complete dissolution). Such a mixture gelled within eight to nine minutes. When leptin was omitted, the gelation time was slightly longer (10-12 minutes). This allowed time to load the syringes.

The delayed gel formulation was injected (while still ungelled) into one group of mice on alternate days at 100 mg/kg/day (2 days worth at 50 mg/kg). A second group was injected with leptin solution (in Tris buffer at 10 mg/mL) daily at 50 mg/kg and a third group received the leptin solution on alternate days at 100 mg/kg. Mice were weighed daily and the weight change was expressed as a percent of the initial weights of the mice.

The alternate day dosing schedule with the delayed gel gave nearly the same weight loss as leptin solution injected daily (8-9%). In contrast, leptin solution administered on alternate days showed a lesser weight loss (6%) of shorter duration. This latter group returned to its baseline weight at 8 days, while the other groups regained their original weights at 10-11 days.

### Example 2

This example shows that production of a buffer-induced calcium alginate delayed gel can lengthen in vitro sustained release in comparison to ungelled formulations. Calcium carbonate suspension was prepared as a 100 mg/mL suspension of a very fine powder. A premix of this with pH 6.7 PIPES buffer was added to a zinc leptin suspension in alginate (Tris buffered at pH 8) that was generated from a concentrated leptin solution (83 mg/mL in Tris pH 8 at the time when zinc was added). One mL of such a mixture was cast in a 10.mL beaker. The final concentrations of all ingredients were: 2% alginate (from autoclaved 10% solution of Keltone LVCR), 15 mM Tris pH8, 50 mg/mL leptin, 1 mM ZnCl2, 10 mM CaCO3 (assuming complete dissolution) and 93 mM PIPES pH 6.7. In the absence of leptin, such a mixture gelled in 7 minutes. After overnight gelation, 0.2 g pieces of the gel were weighed and placed in scintillation vials for release, on the 37°C shaker. This release was compared with the release from the same formulation without calcium and PIPES (no calcium alginate gel). When the zinc leptin alginate thickened formulation (ungelled) was released, the 5 hour timepoint was 75% and there was gradual release to 90% over the following~ 3 days. However, the zinc leptin in the calcium alginate delayed gel exhibited a much more sustained release-35% at 5 hours, 60% at one day, then more gradual release to 70% at 5 days.

### Example 3

This example shows that a δ-gluconolactone-induced gel containing leptin as a fine zinc precipitate, sustains release of leptin. Leptin (~14 mg/mL in Tris pH 8) was added to a pH 6.7 PIPES solution and a ZnCl2 solution was immediately mixed in, rapidly followed by alginate solution (autoclaved 10%) such that the final concentrations of the ingredients were 1.1 mM ZnC12, 2.2% alginate, 10 mM Tris (pH 8) and 22 mM PIPES (pH 6.7). This mixture was concentrated by centrifugation until the leptin level was 46 mg/mL. The gel mixture is then made by stirring in CaCO3 suspension (fine powder) rapidly followed by δ-gluconolactone solution. Final concentrations of the components were 2% alginate, 20 mM PIPES, 9 mM Tris, 1 mM ZnCl2, 16 mM (assuming complete dissolution) CaCO3 and 79 mM δ-gluconolactone. The mixture was drawn into syringes before gelation, and gelation occurred in the syringes after 10 minutes. After overnight storage(3 hours at room temperature and then at 4°C), the gels were injected into mice. Weight loss was monitored in comparison to the buffer control.

Five days worth of leptin was injected at 50 mg/kg/day, i.e., a 250 mg/kg bolus in the gel, and compared to the same bolus of free leptin in solution. The free leptin bolus showed only a modest weight loss maximum of 4.4-4.8% at 2 to 3 days and started gaining weight on day 5. By contrast, at 2 to 4 days, the zinc leptin in the gel produced a 9% weight loss. At five days, the weight loss for the gel was still at 5%, and it remained above baseline (maintained weight loss) when the experiment ended at 7 days.

### Example 4

This example shows that if the protein concentration of alginate-leptin-zinc mixture is sufficiently high, the mixture forms a gel in the absence of calcium that exhibits sustained release. Leptin (~14 mg/mL in Tris pH 8) was added to a buffer solution and a ZnCl2 solution was immediately mixed in, rapidly followed by alginate solution (autoclaved 10%) such that the final concentrations of the ingredients were 1.1 mM ZnCl2, either 1.1 or 2.2% alginate, 10 mM Tris (pH 8) and 22 mM PIPES pH 6.7(if present). This mixture was concentrated by centrifugation until the desired concentration of leptin solids was reached. The ~50 mg/mL formulation had PIPES and 2.2% alginate. The ~100 mg/mL formulation had no PIPES and 1.1% alginate.

Final concentrations of the components for the 50 mg/mL calcium gel of Example 3 were 2% alginate, 20 mM PIPES, 9 mM Tris, 1 mM ZnCl2, 16 mM (assuming complete dissolution) CaCO3 and 79 mM δ-gluconolactone. For the 100 mg/mL calcium gel, the formulation was similar except that the final concentrations of the components were 1% alginate, PIPES was omitted, 13 mM CaCO3 and 67 mM δ-gluconolactone.

For the gels with calcium, the mixtures were drawn into syringes before gelation, and gelation occurred in the syringes after 10 minutes. Where no calcium was included in the gel, the Zn-leptin-alginate mixture was drawn up in syringes and allowed to gel. It appeared that the 50 mg/mL formulation without calcium did not gel. After overnight storage (3 hours at room temperature and then at 4°C), the gels were injected into mice. Weight loss was monitored in comparison to the buffer control.

Five days worth of leptin was injected at 50 mg/kg/day, i.e., a 250 mg/kg bolus in the gel. The calcium gels of Example 3 produced a 9% weight loss within 2-4 days; at five days, the weight loss for the gel was still at 5%, and it remained above baseline (maintained weight loss) when the experiment ended at 7 days. In comparison, the 50 mg/mL formulation without calcium produced a 3% weight loss over days 2 to 4, however, weight loss fell to zero on day 5. By contrast, the 100 mg/mL leptin formulation without calcium was at least as active as the 100 mg/mL leptin formulation with calcium. The peak weight loss for the no-calcium, 100 mg/mL gel was 9% at day 4, the peak weight loss for the calcium, 100 mg/mL gel was 6%, also on day 4. Both of the formulations began gaining weight on day 9.

### Example 5

This example shows that in vitro sustained release of leptin from an alginate delayed gel can be prepared without first forming a leptin-zinc fine precipitate. Leptin( 100 mg/mL; 10 mM Tris HCl, pH 8.8; pH adjusted from 8.0 to 8.8 with 1M NaOH) and 6% alginate( 10 mM Tris HCl, pH 8.6) were cooled on an ice bath. Leptin (0.5 mL) was added to the 6% alginate (0.18 mL) and the mixture stirred on an ice bath for 10-15 minutes; the final pH was 8.6-8.8. To this mixture was added a suspension of 1M CaCO3 (16 mcL) and the resultant suspension stirred well. To this suspension was dropwise added, with stirring, a solution of 0.1 M ZnCl2(100 mcL); water was then added to bring the volume to 1 mL. The suspension mixture was mixed completely and kept on an ice bath for 10-20 minutes. Then a solution of 1.68M δ-gluconolactone ( 56 mcL) was thoroughly stirred into this mixture. An amount of 0.1 mL of the final mixture (50 mg/mL leptin, 1% alginate) was cast on the inside of an eppendorf tube and left overnight at 4C.

After overnight storage an in vitro release was conducted in 10 mM histidine, pH 7.4. The cast gel exhibited little burst and fairly constant leptin release with 50% released in 6 days.

### Example 6

This example shows that a δ-gluconolactone-induced gel containing leptin as a fine zinc precipitate, produces more sustained release of leptin than the same zinc suspension in alginate that is not gelled. The gel with the zinc leptin was prepared as in Example 3. An ungelled zinc leptin suspension in alginate was prepared the same way, except the CaCO₃ and δ-gluconolactone were omitted. Mice were dosed with 250 mg/kg bolus injections and the weight loss experiment was done as described in Example 3. The ungelled suspension produced only a 3% weight loss at days 2-4, while the gelled suspension brought about a 9% weight loss during the same period. Weight loss for the ungelled suspension returned to baseline on day 5, while weight loss for the gelled suspension remained above baseline at seven days, when the experiment ended.

### Example 7

This example shows zero-order release kinetics in a pharmacokinetic/pharmacodynamic study in male rats, demonstrating both sustained release and a simultaneous sustained effect of leptin (i.e., weight loss) delivered by the gel composition described in Example 3. The leptin concentration was 47 mg/mL, and was pre-gelled in the syringe as described in Example 3. Rats were given a bolus dose of 0 mg/kg (control), 50 mg/kg and 250 mg/kg, then blood levels and weight loss were monitored for six days. Leptin-zinc precipitate without the gel was also injected into rats at a dose of 100 mg/kg.

The high dose leptin gel group exhibited a steady blood level of ~2000 ng/mL throughout the period, while the lower dose leptin gel group had a level of ~1/5 that level for four days. In contrast, the leptin without the gel group exhibited a blood level of 2300 ng/mL that peaked at 12 hours and then decreased by a factor of 100 over the same time period. The rats' weight (vs vehicle control) decreased steadily over this period for the high dose leptin gel group. The weight of the lower dose leptin gel group followed the same course for nearly 5 days; at that point, the leptin blood level of the lower dose group declined.

Bioavailability of the leptin drug was assessed by comparing dose-normalized areas under the curve of the leptin gel formulation, leptin without gel formulation and leptin administered intravenously. The bioavailability of the leptin gel formulation was 80% compared to a 63% bioavailability of the leptin without gel formulation.

### Example 8

This example shows the in vitro sustained release of G-CSF from delayed gel vehicles. Both "cast" and "extruded" materials are exemplified. G-CSF in pH 3.4 water was mixed with concentrated alginate (10%) to form a hazy, viscous suspension. This mixture was gelled with CaCO₃ and δ-gluconolactone, at 16 mM and 79 mM, respectively. Before gelation aliquots of the material were either cast in tubes or drawn up in syringes. After 1 hour at room temperature the gels were stored at 4°C (overnight). Before performing the release study, the gel in the syringes were extruded into tubes and allowed to set for 20 minutes. Release buffer was then added. The extruded gel exhibited GCSF release over a three day period, i.e., 11% at 1 hour, 35% at one day, 75% at two days and ~90% at three days. The cast gel released 22% at 1 hour, 80% at 1 day and 94% at two days.

### Example 9

This example demonstrates in vitro sustained release of leptin from an alginate delayed gel prepared from calcium salt (CaSO₄) without the addition of a proton donor. Unbuffered leptin at pH 8 was used to form a zinc-leptin suspension in 2% alginate. The final concentrations of ZnCl₂ and leptin were 1 mM and 55 mg/mL, respectively. A fine powder of CaSO₄ was mixed with the zinc-leptin-alginate suspension such that the final mixture was 10 mM in CaSO₄. Aliquots of the material were cast on the walls of eppendorf tubes. The mixture gelled in 4-5 minutes.

After overnight storage at room temperature, a release study was conducted. The protein release from the gel exhibited a low burst (≤ 5%) at 1 hour. At one day 11% of the leptin was released. Leptin release was gradual, 1.1% per day, for the next seven days.

### Example 10

This example demonstrates in vitro sustained release of leptin from an alginate delayed gel prepared from a calcium salt in a nonaqueous solvent without the addition of a proton donor. A lyophilized powder of leptin is suspended in 2% alginate in dry dimethyl sulfoxide. A fine powder of calcium oleate is mixed with the alginate-leptin suspension such that the final mixture is 10 mM in calcium. Aliquots of the material are cast on the walls of test tubes. The mixture gels over time.

After overnight storage at room temperature, a release study is conducted. The protein release from the gel is gradual and sustained, over the next 7 days.

### Example 11

This example demonstrates in vivo sustained release of leptin from an alginate gel prepared from a calcium salt as described in Example 10. A weight loss study in mice is performed after a single injection of this leptin-containing gel at 250 mg/kg. Weight loss is measured over several days.

### Example 12

This example demonstrates in vitro sustained release of G-CSF from an alginate delayed gel prepared from a calcium salt in a nonaqueous solvent without the addition of a proton donor. A lyophilized powder of G-CSF is suspended in 2% alginate in dry dimethyl sulfoxide. A fine powder of calcium oleate is mixed with the alginate-G-CSF suspension such that the final mixture is 10 mM in calcium. Aliquots of the material are cast on the walls of test tubes. The mixture gels over time.

After overnight storage at room temperature, a release study is conducted. The protein release is gradual and sustained.

### Example 13

This example shows in vitro sustained release of consensus interferon, as disclosed in U.S. Patent No. 4,695,623, supra, from alginate delayed gels. Water, ZnCl₂, Tris buffer and consensus interferon (in 10 mM Tris pH 7.5) were mixed with alginate solution, then mixed with CaCO₃ and δ-gluconolactone, such that the final concentrations of the components were 1 mg/mL consensus interferon, 10 mM ZnCl₂, 1% alginate, 20 mM Tris, 10 mM CaCO₃ and 40 mM δ-gluconolactone. The mixture was cast on an eppendorf tube (0.4 mL per tube), gelled at room temperature and stored overnight at 4°C. After overnight storage an in vitro release was conducted in 10 mM histidine buffer, pH 7.4. The cast gel exhibited little initial burst-the percent release was 3% at 1 hour, 14% at one day. By 4 days 70% had released. At 5-6 days, the release rate slowed to <5% per day.

### Example 14

This example shows that alginate delayed gels can be used for sustained release of consensus interferon. An alginate consensus interferon delayed gel was prepared in accordance with Example 13 except that final concentrations were as follows: 0.2 mg/mL consensus interferon, 10 mM ZnCl₂, 2% alginate, 20 mM Tris, 10 mM CaCO₃ and 40 mM δ-gluconolactone. Another formulation was prepared with the same composition, except the consensus interferon final concentration was 1 mg/mL. The mixtures were drawn up in syringes and gelled after 2 hours. The 0.2 mg/mL formulation was injected subcutaneously at 1 mg/kg and the 1 mg/mL formulation at 1 mg/kg and 5 mg/kg doses in male Syrian hamsters. Blood was collected by cardiac puncture and assayed for consensus interferon to observe sustained release of the drug.

## Claims

1. A pharmaceutical formulation of a sustained-released gel composition comprising:
a) alginate;
b) a biologically active agent; and
c) at least one bound polyvalent metal ion,
wherein the gel forms in a time-delayed manner, in a pharmaceutically acceptable carrier or diluent.

2. A pharmaceutical formulation according to Claim 1 wherein the sustained-release composition further comprises:
d) least one proton donor capable of generating an acid via hydrolysis, poor solubility or slow dissolution, whereby the bound polyvalent metal ion is released

3. A pharmaceutical formulation according to claim 2, wherein the proton donor is selected from buffers, esters, poorly soluble acids, slowly dissolving acids or lactones.

4. A pharmaceutical formulation according to Claim 3, wherein the proton donor is gluconolactone

5. A pharmaceutical formulation according to any one of the preceding claims, wherein the bound polyvalent metal ion is a mixture of bound and unbound polyvalent metal ion.

6. A pharmaceutical formulation according to any one of the preceding claims, further comprising excipient(s) for stabilizing the biologically active agent or alginate.

7. A pharmaceutical formulation according to any one of the preceding claims, wherein the bound polyvalent metal ion is a salt selected from phosphates, tartrates, sulfates, carbonates, hydroxides or fatty acid anions thereof.

8. A pharmaceutical formulation according to Claim 7, wherein the metal ion is selected from manganese, strontium, iron, magnesium, calcium, barium, copper, aluminium or zinc.

9. A pharmaceutical formulation according to Claim 8, wherein the metal ion is calcuim.

10. A pharmaceutical formulation according to any one of the preceding claims, wherein the alginate contains at least 30% guluronic acid.

11. A pharmaceutical formulation according to any one of the preceding claims, wherein the biologically active agent comprises a protein.

12. A pharmaceutical formulation according to Claim 11, wherein the protein is selected from hematopoetic factors, colony stimulating factors, anti-obesity factors, growth factors, and antiinflamatory factors.

13. A pharmaceutical formulation according to Claims 11 or 12 wherein the protein is selected from leptin, G-CSF, SCF, BDNF, GDNF, NT3, GM-CSF, IL-1ra, IL2, TNF-bp, MGDF, OPG, interferons, erythropoietin, KGF, insulin and analogs or derivatives thereof.

14. A pharmaceutical formulation according to any one of Claims 1 to 10, wherein the biologically active agent is a complexed biologically active agent.

15. A pharmaceutical formulation according to Claim 14, wherein the complexed biologically active agent is a precipitated protein.

16. A pharmaceutical formulation according to 15, wherein the precipitated protein is a zinc leptin precipitate.

17. A pharmaceutical formulation according to any one of the preceding claims, wherein the formulation is in a syringe

18. The use of a sustained-release composition according to Claims 1 to 16 in a pharmaceutically acceptable carrier, diluent or adjuvant for the manufacture of a medicament for treating an indication.

19. A use according Claim 18, wherein the indication is disorder selected from excess weight, diabetes, high blood lipid level, artherial sclerosis, artherial plaque, the reduction or prevention of gall stones formation, insufficient lean tissue mass, insufficient sensitivity to insulin, and stroke.

20. A use according to Claim 18, wherein the indication is a disorder selected from hematopoietic cell deficiencies, infection, and neutropenia.

21. A use according to Claim 18, wherein the indication is inflamation.

## Patentansprüche

1. Pharmazeutische Formulierung einer nachhaltig wirkenden Gelzusammensetzung umfassend:
a) Alginat;
b) ein biologisch aktives Agens; und
c) wenigstens ein gebundenes polyvalentes Metallion,
wobei sich das Gel zeitverzögert ausbildet, in einem pharmazeutisch akzeptablen Träger oder Verdünnungsmittel.

2. Pharmazeutische Formulierung nach Anspruch 1, wobei die nachhaltig wirkende Zusammensetzung weiter umfasst:
d) wenigstens einen Protonendonor, der in der Lage ist, vermittels Hydrolyse, geringer Löslichkeit oder langsamen Auflösens eine Säure zu erzeugen, wobei das gebundene polyvalente Metallion freigesetzt wird.

3. Pharmazeutische Formulierung nach Anspruch 2, wobei der Protonendonor ausgewählt ist aus Puffern, Estern, schwach löslichen Säuren, langsam in Lösung gehenden Säuren oder Laktonen.

4. Pharmazeutische Formulierung nach Anspruch 3, wobei der Protonendonor Gluconolakton ist.

5. Pharmazeutische Formulierung nach einem der vorhergehenden Ansprüche, wobei das gebundene polyvalente Metallion eine Mischung aus gebundenem und nicht gebundenem polyvalenten Metallion ist.

6. Pharmazeutische Formulierung nach einem der vorhergehenden Ansprüche, weiter umfassend Bindemittel zum Stabilisieren des biologisch aktiven Agens oder Alginats.

7. Pharmazeutische Formulierung nach einem der vorhergehenden Ansprüche, wobei das gebundene polyvalente Metallion ein Salz ist, das ausgewählt ist aus Phosphaten, Tartraten, Sulfaten, Carbonaten, Hydroxiden oder Fettsäureanionen davon.

8. Pharmazeutische Formulierung nach Anspruch 7, wobei das Metallion ausgewählt ist aus Mangan, Strontium, Eisen, Magnesium, Kalzium, Barium, Kupfer, Aluminium oder Zink.

9. Pharmazeutische Formulierung nach Anspruch 8, wobei das Metallion Kalzium ist.

10. Pharmazeutische Formulierung nach einem der vorhergehenden Ansprüche, wobei das Alginat wenigstens 30 % Glucuronsäure enthält.

11. Pharmazeutische Formulierung nach einem der vorhergehenden Ansprüche, wobei das biologisch aktive Agens ein Protein umfasst.

12. Pharmazeutische Formulierung nach Anspruch 11, wobei das Protein ausgewählt ist aus hämatopoetischen Faktoren, koloniestimulierenden Faktoren, Faktoren gegen Fettleibigkeit, Wachstumsfaktoren und anti-entzündlichen Faktoren.

13. Pharmazeutische Formulierung nach den Ansprüchen 11 oder 12, wobei das Protein ausgewählt ist aus Leptin, G-CSF, SCF, BDNF, GDNF, NT3, GM-CSF, IL-1ra, IL2, TNF-bp, MGDF, OPG, Interferonen, Erythropoietin, KGF, Insulin und Analogen oder Derivaten davon.

14. Pharmazeutische Formulierung nach einem der Ansprüche 1 bis 10, wobei das biologisch aktive Agens ein komplexiertes biologisch aktives Agens ist.

15. Pharmazeutische Formulierung nach Anspruch 14, wobei das komplexierte biologisch aktive Agens ein präzipitiertes Protein ist.

16. Pharmazeutische Formulierung nach Anspruch 15, wobei das präzipitierte Protein ein Zink-Leptin-Präzipitat ist.

17. Pharmazeutische Formulierung nach einem der vorhergehenden Ansprüche, wobei die Formulierung sich in einer Spritze befindet.

18. Verwendung einer nachhaltig wirkenden Zusammensetzung gemäß den Ansprüchen 1 bis 16 in einem pharmazeutisch akzeptablen Träger, Verdünnungsmittel oder Adjuvans für die Herstellung eines Medikaments zur Behandlung einer Indikation.

19. Verwendung nach Anspruch 18, wobei die Indikation eine Störung ist, die ausgewählt ist aus Übergewicht, Diabetes, hohe Blutlipidtiter, Arteriosklerose, Arterioskleroseherd, die Verringerung oder Verhinderung von Gallensteinbildung, ungenügende fettfreie Gewebemasse, ungenügende Empfindlichkeit gegenüber Insulin, und Schlaganfall.

20. Verwendung nach Anspruch 18, wobei die Indikation eine Störung ist, die ausgewählt ist aus Mangel an hämatopoetischen Zellen, Infektion und Neutropenie.

21. Verwendung nach Anspruch 18, wobei die Indikation Entzündung ist.

## Revendications

1. Préparation pharmaceutique d'une composition de gel à libération prolongée comprenant :
a) un alginate ;
b) un agent biologiquement actif ; et
c) au moins un ion métallique polyvalent lié,
dans laquelle le gel se forme de manière différée, dans un support ou diluant pharmaceutiquement acceptable.

2. Préparation pharmaceutique selon la revendication 1, dans laquelle la composition à libération prolongée comprend en outre :
d) au moins un donneur de proton capable de produite un acide par hydrolyse, faible solubilité ou dissolution lente, moyennant quoi l'ion métallique polyvalent lié est libéré.

3. Préparation pharmaceutique selon la revendication 2, dans laquelle le donneur de proton est choisi parmi des tampons, des esters, des acides faiblement solubles, des acides à dissolution lente ou des lactones.

4. Préparation pharmaceutique selon la revendication 3, dans laquelle le donneur de proton est la gluconolactone.

5. Préparation pharmaceutique selon l'une quelconque des revendications précédentes, dans laquelle l'ion métallique polyvalent lié est un mélange d'ion métallique polyvalent lié et non lié.

6. Préparation pharmaceutique selon l'une quelconque des revendications précédentes, comprenant en outre un ou des excipients pour stabiliser l'agent biologiquement actif ou l'alginate.

7. Préparation pharmaceutique selon l'une quelconque des revendications précédentes, dans laquelle l'ion métallique polyvalent lié est un sel choisi parmi les phosphates, tartrates, sulfates, carbonates, hydroxydes ou des anions d'acides gras de ceux-ci.

8. Préparation pharmaceutique selon la revendication 7, dans laquelle l'ion métallique est choisi parmi le manganèse, le strontium, le fer, le magnésium, le calcium, le baryum, le cuivre, l'aluminium ou le zinc.

9. Préparation pharmaceutique selon la revendication 8, dans laquelle l'ion métallique est du calcium.

10. Préparation pharmaceutique selon l'une quelconque des revendications précédentes, dans laquelle l'alginate contient au moins 30 % d'acide guluronique.

11. Préparation pharmaceutique selon l'une quelconque des revendications précédentes, dans laquelle l'agent biologiquement actif comprend une protéine.

12. Préparation pharmaceutique selon la revendication 11, dans laquelle la protéine est choisie parmi les facteurs hématopoïétiques, les facteurs stimulant les colonies, les facteurs anti-obésité, tes facteurs de croissance et les facteurs anti-inflammatoires.

13. Préparation pharmaceutique selon les revendications 11 ou 12, dans laquelle la protéine est choisie parmi leptine, G-CSF, SCF, BDNF, GDNF, NT3, GM-CSF, IL-1ra, IL2, TNF-bp, MGDF, OPG, interférons, érythropoïétine, KGF, insuline et des analogues ou dérivés de ceux-ci.

14. Préparation pharmaceutique selon l'une quelconque des revendications 1 à 10, dans laquelle l'agent biologiquement actif est un agent biologiquement actif complexé.

15. Préparation pharmaceutique selon la revendication 14, dans laquelle l'agent biologiquement actif complexé est une protéine précipitée.

16. Préparation pharmaceutique selon la revendication 15, dans laquelle la protéine précipitée est un précipité de leptine-zinc.

17. Préparation pharmaceutique selon l'une quelconque des revendications précédentes, dans laquelle la préparation se trouve dans une seringue.

18. Utilisation d'une composition à libération prolongée selon les revendications 1 à 16 dans un support, diluant ou adjuvant pharmaceutiquement acceptable, pour la fabrication d'un médicament destiné à traiter une indication.

19. Utilisation selon la revendication 18, dans laquelle l'indication est un trouble choisi parmi l'excès de poids, le diabète, l'hyperlipidémie, l'artériosclérose, la plaque artérielle, la réduction ou la prévention de la formation de calculs biliaires, une masse tissulaire maigre insuffisante, une sensibilité à l'insuline insuffisante et un accident vasculaire cérébral.

20. Utilisation selon la revendication 18, dans laquelle l'indication est un trouble choisi parmi des déficits en cellules hématopoïétiques, une infection et une neutropénie.

21. Utilisation selon la revendication 18, dans laquelle l'indication est une inflammation,
